## Europäisches Patentamt
## European Patent Office
## Office européen des brevets

(19)

(11) Veröffentlichungsnummer: **0 001 291**
**B1**

(12)

# EUROPÄISCHE PATENTSCHRIFT

(45) Veröffentlichungstag der Patentschrift:
07.01.81

(51) Int. Cl.³: **C 07 D 307/08**

(21) Anmeldenummer: **78100974.1**

(22) Anmeldetag: **25.09.78**

(54) **Verfahren zur Herstellung von Tetrahydrofuran aus Butandiol-1,4.**

(30) Priorität: **27.09.77 DE 2743345**

(43) Veröffentlichungstag der Anmeldung:
**04.04.79 Patentblatt 79/7**

(45) Bekanntmachung des Hinweises auf die Patenterteilung:
**07.01.81 Patentblatt 81/1**

(84) Benannte Vertragsstaaten:
**BE DE FR GB NL**

(56) Entgegenhaltungen:
**DE-C-711 709**
**FR-A-2 295 036**
**FR-A-2 345 440**

(73) Patentinhaber: **BASF Aktiengesellschaft,
Carl-Bosch-Strasse 38, D-6700 Ludwigshafen (DE)**

(72) Erfinder: **Mueller, Herbert, Dr., Carostrasse 53,
D-6710 Frankenthal (DE)**
Erfinder: **Huchler, Otto Hermann, Dr., Weinbietstrasse 38,
D-6703 Limburgerhof (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).

Verfahren zur Herstellung von Tetrahydrofuran aus Butandiol-1,4

Die Erfindung betrifft ein Verfahren zur Herstellung von Tetrahydrofuran durch Dehydratisierung von Butandiol-1,4 in Gegenwart von Bleicherden.

Es sind bereits zahlreiche Verfahren der Dehydratisierung von Butandiol-1,4 zu Tetrahydrofuran (THF) bekannt geworden. Der Stand der Technik bis 1955 ist in Liebigs Annalen der Chemie, Band 596, Seite 81 (1955) beschrieben worden.

Als wasserabspaltende Mittel werden Phosphorsäure, Schwefelsäure, Oxalsäure, Kupfersulfat, Magnesiumchlorid, Zinkchlorid und viele andere genannt. Mit diesen sauer reagierenden Verbindungen wird die Wasserabspaltung gewöhnlich in flüssiger Phase bei Temperaturen bis zu 250 °C durchgeführt.

Arbeitet man bei Temperaturen über 250 °C, so werden auch gewisse andere Katalysatoren, z.B. Oxide des Aluminiums, Titans, Zirkons, Wolframs, ferner Bleicherden und Phosphate wirksam. Da Butandiol bei 245 °C siedet, führt man die Umsetzung dieses Stoffes entweder in der Gasphase durch oder man arbeitet unter Druck (vergleiche DE-PS 711 709). Bei den relativ hohen Reaktionstemperaturen beobachtet man aber schon in merklichem Umfange Butadienbildung.

Die Vielzahl der bekannt gewordenen Verfahren besitzt jedoch eine Reihe von Nachteilen: Bei löslichen Katalysatoren tritt ein merklicher Katalysatorverbrauch ein, und die Ausbeute übersteigt 90% im allgemeinen nicht. Auch später bekannt gewordene, verbesserte Verfahren, z.B. nach der DE-PS 850 750, wobei die Umsetzung mittels eines Kationenaustauschers durchgeführt wird, bringen insbesondere keine wesentliche Verbesserung, zumal die handelsüblichen Austauscherharze bei Reaktionstemperaturen von 150 °C nur eine geringe Haltbarkeit besitzen. Die Verwendung geringer Mengen konzentrierter Schwefelsäure, wie dies in der DE-PS 1 043 342 beschrieben ist, erfordert die Verwendung von Syntheseapparaten mit korrosionsfester Innenauskleidung und ist deshalb relativ teuer. Ausserdem werden erfahrungsgemäss auch nach dieser Methode kaum mehr als 10 000 Teile Butandiol je Gewichtsteil Schwefelsäure umgesetzt: danach ist der Katalysator durch Verunreinigungen verbraucht und muss vernichtet werden. Dabei gehen auch die im Reaktionsapparat noch vorhandenen Mengen an Butandiol verloren. Angesichts der Tatsache, dass Äther wie THF Grossprodukte sind, bedeutet die Menge des zu vernichtenden Katalysators sowie die dabei verlorengehende Menge des Ausgangsproduktes einen erheblichen Nachteil.

Auf die Möglichkeit, zur Herstellung von Tetrahydrofuran Bleicherden zu verwenden, wurde wiederholt hingewiesen (Liebigs Annalen a.a.O. oder z.B. DE-PS 700 036). Eine genaue Verfahrensbeschreibung mit Bleicherde als Katalysator ist jedoch nicht bekannt. Lediglich ein Verfahren zur Herstellung von THF mit Hilfe von Aluminium- und Magnesiumsilikaten wird in der Deutschen Offenlegungsschrift 2 461 922 beschrieben. Es handelt sich dabei um einen Gasphasenprozess. Gegenüber der Arbeitsweise in der flüssigen Phase weist er unter anderem den Nachteil auf, dass das Butandiol, das umgesetzt werden soll, vor der eigentlichen Reaktion verdampft werden muss. Ein solches Verfahren ist natürlich vom energetischen Standpunkt gesehen ungünstig. Wird in diesem Prozess Butandiol minderer Qualität eingesetzt oder ein Rohprodukt, so ist die Lebensdauer der Katalysatoren gering. Durch Ablagerung von polymeren Produkten verlieren die Katalysatoren auch hier sehr schnell an Aktivität.

Bei unseren Untersuchungen zur Verwendung von Bleicherde zur Herstellung von THF stellten wir fest, dass diese sehr aktive Dehydratisierungskatalysatoren sind, Butandiol-1,4 mit hoher Geschwindigkeit bei verhältnismässig tiefen Temperaturen in THF überführen.

Als schwerwiegender Nachteil ergab es sich daher, dass auch sie geringe Mengen des Butandiols (ca. 0,5%) in ein hochmolekulares, unlösliches Polymerisat überführen.

Da die Bleicherden normalerweise nur als feine Pulver oder als Granulat mit kleiner Korngrösse zu erhalten sind, muss man sie in suspendierter Form anwenden. Dies führt verfahrenstechnisch zu einer Anordnung, bei der Rührkessel oder ähnliche Reaktionsbehälter verwendet werden, in denen sich das Polymerisat anreichert und in fester Form an den Wandungen und Heizungen ablagert. Ebenso verliert der Katalysator sehr schnell an Aktivität. Der Katalysator und das in allen gebräuchlichen Lösungsmitteln unlösliche Polymerisat muss mechanisch entfernt werden. Eine solche Massnahme ist natürlich aufwendig und mit hohen Produktionsausfallzeiten verbunden, um so mehr, wenn es gilt, technisch übliche, nicht besonders reine Qualitäten von Butandiol-1,4 in THF zu überführen. Denn die Entstehung von Polymeren stellt man besonders ausgeprägt fest, wenn technisches Butandiol-1,4 verwendet wird. Möglicherweise sind die im Butandiol vorhandenen Verunreinigungen für die Entstehung des Polymerisates verantwortlich.

Es war deshalb eine Aufgabe der Erfindung, ein Verfahren anzugeben bzw. einen Katalysator auf der Grundlage von Bleicherden zu finden, mit dem die Umsetzung von Butandiol-1,4 zu Tetrahydrofuran störungsfrei mit hoher Ausbeute und mit geringem Katalysatorverbrauch gelingt, wobei besonders darauf zu achten war, dass auch das technisch übliche, nicht besonders reine Butandiol keine Störungen verursachen sollte. Insbesondere war auch Wert darauf zu legen, dass rohe, wässrige Butandiollösungen, die bei der Hydrierung von nach dem Reppe-Verfahren gewonnenen Butandiol entsteht, mit dem Katalysator umgesetzt werden kann. Es ist bekannt, dass undestilliertes Butandiol aus dieser Quelle durch Natrium- oder Calciumformiat verunreinigt sein kann. Für die Überführung eines solchen Produktes in THF beschreibt die Patentschrift DE-OS 2 303 619 ein verhältnismässig aufwendiges und teures Verfahren, in dem mit Hilfe von Tallöl die Verunreinigungen aus dem Reaktionssystem ausgeschleust werden.

Katalysatoren, die diese Aufgabe erfüllen, sind Bleicherden, die erfindungsgemäss zusammen mit Alkali- oder Erdalkalimetallcarbonaten bzw. Bicarbonaten verwendet werden. Bleicherden, auch Fullererden genannt, sind kolloidale, wasserhaltige Aluminiumhydrosilikate aus der Montmorillonitgruppe, bei denen die Aluminiumionen teilweise durch Eisen oder Magnesiumionen ersetzt sein können. Das Verhältnis von Kieselsäure zu Tonerde in diesen Mineralien beträgt etwa 4:1. Es sind handelsübliche Produkte, die i.a. durch Säurebehandlung aktiviert sind und in grossem Umfange zur Raffination von Speiseölen und Fetten sowie Mineralölen verwendet werden.

Verwendet man Bleicherde ohne Alkali- oder Erdalkalimetallcarbonat für die Herstellung von THF aus Butandiol-1,4, so entsteht zwar in der Anfangsphase der Umsetzung der cyclische Äther mit hoher Reaktionsgeschwindigkeit, die aber bereits nach 1 bis 2 Tagen auf einen Bruchteil der ursprünglichen absinkt. Ausserdem bildet sich das bereits erwähnte unlösliche Polymerisat. Im Falle der Verwendung von Bleicherde zusammen mit dem Carbonat entsteht hingegen kein unlösliches Polymerisat. Lediglich im Butandiol vorhandene Verunreinigungen sammeln sich im Suspensionbetrieb als Sumpf an. Sie können sehr leicht durch Waschen mit einem Lösungsmittel, beispielsweise Methanol, vom Katalysator abgetrennt werden.

Die Menge an Alkali- oder Erdalkalimetallcarbonat bzw. -bicarbonat, die zugesetzt wird, beträgt 0,3 bis 1,8, vorteilhaft 0,5 bis 1,5 Gewichtsprozent, bezogen auf das Gewicht des Katalysators.

Nach der Erfindung wird die Wasserabspaltung in flüssiger Phase mit suspendiertem oder fest angeordnetem Katalysator vorgenommen, d.h. im letzteren Falle ist Bleicherde in geeigneter Form fest angeordnet. Das Carbonat kann dem Reaktionsgemisch als Feststoff oder in Lösung zugesetzt werden.

Das neue Verfahren liefert praktisch quantitative Ausbeute, besitzt eine beliebig hohe Katalysatorproduktivität und ist umweltfreundlich. Da das Reaktionsgemisch keine korrosiven Substanzen enthält, lassen sich Produktionsapparate aus billigen Werkstoffen, wie beispielsweise gewöhnlicher Apparatestahl, verwenden. Auf die Anwendung verbleiter Behälter, die bei der Durchführung der Reaktion mit Schwefelsäure nötig und technisch gebräuchlich sind, kann verzichtet werden.

Die Reaktionsgeschwindigkeit hängt ab von der gewählten Reaktionstemperatur und der Katalysatormenge. Bei 200 °C z.B. gelingt es, je Gewichtsteil Katalysator bis zu 20 Teile THF je Stunde aus Butandiol herzustellen. Man führt die Dehydratisierung zwischen 140 und 230, bevorzugt zwischen 150 und 200 °C durch. Im allgemeinen arbeitet man bei gewöhnlichem, d.h. atmosphärischem oder geringfügig höherem oder niedrigerem Druck. Bei vermindertem Druck ist darauf zu achten, dass die Siedetemperatur des Butandiols dabei die Reaktionstemperatur nicht unterschreitet. Die angewandte Katalysatormenge ist nicht von chemischen Umständen, sondern von technischen Gegebenheiten abhängig und richtet sich z.B. nach der erzielbaren Destillationsgeschwindigkeit, der Reaktorgeometrie und dergleichen. Im allgemeinen wird der Katalysator als 0,5- bis 10-gewichtsprozentige Suspension verwendet.

Die Dehydratisierung kann zur Stabilisierung der Reaktionstemperatur auch in Gegenwart eines chemisch indifferenten Lösungsmittels durchgeführt werden. Geeignete Verdünnungsmittel sind z.B. Kohlenwasserstoffe, wie Benzinfraktionen, hochsiedende Ketone oder Äther, wie Dodecan und Decanon oder Dihexyläther und bevorzugt das Reaktionsprodukt selbst, wobei allerdings wegen dessen üblicher Flüchtigkeit erhöhter Druck anzuwenden wäre. Meist kann man auf die Verwendung eines Verdünnungsmittels verzichten. Wasser kommt jedenfalls in untergeordneter Menge als Lösungsmittel ebenfalls in Betracht, zumal es während der Reaktion entsteht.

Das Verfahren kann zwar absatzweise betrieben werden, jedoch ist die fortlaufende Betriebsweise von Vorteil. Besonders zweckmässig wird das umzusetzende Butandiol in das Reaktionsgemisch unter Rühren in dem Masse zugegeben, in dem das Reaktionsprodukt aus dem Reaktionsgefäss z.B. über eine entsprechende Fraktionierkolonne abdestilliert. In diesem Falle erhält man am Kolonnenkopf in der Regel das reine Produkt zusammen mit dem gebildeten und gegebenenfalls dem mit dem Rohprodukt zugeführten Wasser.

Die in dem folgenden Beispiel genannten Teile sind Gewichtsteile. Sie verhalten sich zu Volumina wie Kilogramm zu Liter.

Beispiel

Man arbeitet in einer Destillationsblase mit Rührwerk und einem Fraktionieraufsatz. Als Katalysator werden 50 Teile einer Bleicherde, die erhältlich ist als Tonsil, Optimum FF® (Hersteller Südchemie, München) und 0,65 Teile Natriumbicarbonat zu 2000 Teilen Butandiol-1,4 hinzugefügt und das Reaktionsgemisch auf 180 °C erwärmt. Bereits bei 150 °C setzt lebhafte Reaktion ein. Bei 180 °C werden stündlich 450 Teile Butandiol zu THF umgesetzt. Im Masse der Umsetzungsgeschwindigkeit wird in der Blase das verbrauchte Butandiol durch neuen Zulauf ergänzt. Nachdem 100 000 Teile Butandiol umgesetzt sind, beträgt die Reaktionsgeschwindigkeit noch 150 Teile pro Stunde. Dem Reaktionssumpf werden dann erneut 50 Teile Tonsil und 0,65 Teile Natriumbicarbonat zugesetzt. Die stündliche Umsatzgeschwindigkeit steigt danach wieder auf ca. 550 Teile Butandiol-1,4 an, und es werden weitere

100 000 Teile Butandiol zu THF umgesetzt. Zum Schluss, nach dem Abstellen des Zulaufs, wird der Sumpf in der Blase weitere 20 Stunden auf 180 °C erwärmt. Danach ist die Reaktion beendet. Als Rückstand verbleiben neben ca. 100 Teilen anorganischer Feststoffe 330 Teile eines öligen Rückstandes, der in Methanol leicht löslich ist. Die Ausbeute ist praktisch quantitativ.

Arbeitet man auf die gleiche Weise, unterlässt aber den Zusatz von Natriumbicarbonat, so wird bereits nach einem Durchsatz von 45 000 Teilen Butandiol eine Umsatzgeschwindigkeit von nur 50 Teilen Butandiol pro Stunde gemessen. Man versetzt erneut mit 50 Teilen Tonsil/Optimum FF. Es stellt sich eine Anfangsumsatzgeschwindigkeit von 500 Teilen Butandiol pro Stunde ein. Nachdem weitere 50 000 Teile Butandiol zur Reaktion gebracht worden sind, sinkt die Umsatzgeschwindigkeit auf nahezu 0. Es bleiben 400 Teile festes unlösliches Polymerisat zurück, das nur mit grosser Mühe aus dem Reaktionsbehälter entfernt werden kann.

Ein ähnliches Ergebnis wie oben beschrieben erhält man, wenn man statt jeweils 0,65 Teilen Natriumbicarbonat 0,3 Teile Natriumcarbonat oder Calciumcarbonat verwendet.

**Patentansprüche**

1. Verfahren zur Herstellung von Tetrahydrofuran durch Dehydratisieren von Butandiol-1,4 in flüssiger Phase an einem dehydratisierend wirkenden Katalysator aus der Gruppe der Bleicherden, dadurch gekennzeichnet, dass man die Umsetzung in Gegenwart von 0,3 bis 1,8 Gewichtsprozent, bezogen auf die angewandte Bleicherdemenge, eines Alkali- oder Erdalkalimetallcarbonats bzw. -bicarbonats vornimmt.

2. Verfahren nach Anspruch 1, dadurch gekennzeichnet, dass man 0,5 bis 1,5 Gewichtsprozent, bezogen auf die angewandte Bleicherdemenge, des Alkali- oder Erdalkalimetallcarbonats bzw. -bicarbonats verwendet.

3. Verfahren nach Anspruch 1, dadurch gekennzeichnet, dass man die Dehydratisierung bei 140 bis 230 °C durchführt.

**Claims**

1. A process for the manufacture of tetrahydrofuran by dehydrating butanediol-1,4 in the liquid phase over a dehydrating catalyst selected from the group of the bleaching earths, characterized in that the reaction is carried out in the presence of from 0.3 to 1.8% by weight, based on the amount of bleaching earth used, of an alkali metal or alkaline earth metal carbonate or bicarbonate.

2. A process as claimed in claim 1, characterized in that from 0.5 to 1.5% by weight, based on the amount of bleaching earth used, of the alkali metal or alkaline earth metal carbonate or bicarbonate is employed.

3. A process as claimed in claim 1, characterized in that the dehydration is carried out at from 140° to 230 °C.

**Revendications**

1. Procédé pour la préparation de tétrahydrofuranne par déshydratation de butane-diol-1,4 en phase liquide sur un catalyseur à activité déshydratante du groupe des terres décolorantes, caractérisé en ce qu'on procède à la transformation en présence de 0,3 à 1,8% en poids, par rapport à la quantité de terre décolorante utilisée, d'un carbonate ou bicarbonate de métal alcalin ou alcalino-terreux.

2. Procédé selon la revendication 1, caractérisé en ce qu'on utilise 0,5 à 1,5% en poids, par rapport à la quantité de terre décolorante utilisée, du carbonate ou bicarbonate de métal alcalin ou alcalino-terreux.

3. Procédé selon la revendication 1, caractérisé en ce qu'on mène la déshydratation à une température de 140 à 230 °C.